# EUROPEAN PATENT APPLICATION

(11) **EP 2 077 109 A1**
(43) Date of publication of application: **08.07.2009**
(21) Application number: 07830635.4
(22) Date of filing: 26.10.2007
(51) Int. Cl.: A61K 31/167, A61K 8/42, A61K 8/49, A61K 31/40, A61K 31/44, A61P 1/02, A61P 31/02, A61P 31/04, A61Q 11/00, C07C 235/80, C07D 213/75, C07D 295/22

(54) **AMIDE COMPOUND, SALT THEREOF, AND BIOFILM REMOVER USING THEM**

(30) Priority: 27.10.2006 JP 2006293349
(71) Applicant: The University of Tokyo, Bunkyo-ku, Tokyo 1138654 (JP); Otsuka Chemical Co., Ltd., Osaka-shi, Osaka 540-0021 (JP)
(72) Inventor: SUGA, Hiroaki, Bunkyo-ku Tokyo 1138654 (JP); IGARASHI, Jun, Tokushima-shi Tokushima 7710193 (JP)
(74) Representative: Barz, Peter
(86) International application number: PCT/JP2007/070901
(87) International publication number: WO 2008/050858

(57) **Abstract**

The present invention provides a biofilm stripping agent for removing biofilms already formed. The biofilm stripping agent of the present invention contains, as an active ingredient, an amide compound or salt thereof denoted by General Formula (1), 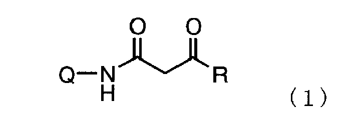 wherein R is a C₁₋₁₁ alkyl group, and Q is a substituent denoted by the following Formulas (Q1), (Q2) or (Q3), 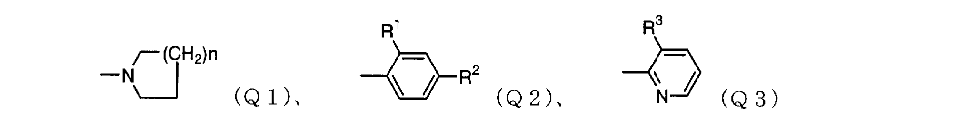 wherein, in Formula (Q1), n is an integer ranging from 0 to 4; in Formula (Q2), R¹ is a C₁₋₄ alkyl group, and R² is a hydroxyl group or carbamoyl group; in Formula (Q3), R³ is a hydrogen atom, hydroxyl group or carbamoyl group.

## Description

### TECHNICAL FIELD

The present invention relates to a biofilm remover (biofilm stripping agent). The present invention further relates to a new amide compound and salt thereof serving as a biofilm remover (biofilm stripping agent).

### BACKGROUND ART

A certain kind of microorganism, such as bacteria or fungus, adheres to a carrier surface and forms a colony thereon. When the colony contains a certain number of bacillus cells, it forms/secretes an organic substance such as polysaccharide or glycoprotein that grows into a biofilm. A biofilm is akin to a medium that allows other microorganisms to enter and form a complicated microorganism group inside. Such biofilm deposits can be found everywhere in the natural environment, in industrial areas, and in humans. Such biofilm deposits cause numerous problems, including those in industrial facilities such as the erosion of metal tubing in factory drain pipes or malfunctions during valve operations, the generation of Legionella bacteria in circulating-type bathtubs, as well as various human infections including skin diseases such as pimples or skin inflammation, eye infections such as microbial keratitis via contact lenses, intraoral diseases such as caries or periodontitis, or other diseases such as otitis media, bacterial prostatitis, or cystic fibrosis pneumonia.

Many of these biofilm infections, for example, periodontitis, are intractable, and one reason for the intractableness is that the microorganisms in a biofilm are covered by a film (an extracellular matrix), and will therefore not directly come into contact with immune system cells or antibacterial substances. Another reason is that bacterias in a biofilm have very slow metabolisms, which is also thought to interfere with antibiotics, which show the greatest effect in actively-dividing cells. For the reasons above, in order to completely cure biofilm infections, it is necessary to ensure both the prevention of biofilm deposits by microorganisms as well as the removal of deposited biofilms.

Biofilms are generally removed physically, for example, by scraping them off with a brush, etc. However, because biofilms generally adhere tightly to a carrier surface, this is not particularly effective, even with a great deal of effort.

In light of such existing problems, compounds that can control the deposit of biofilms have been attracting attention. For example, Patent Document 1 discloses the invention of a compound having a certain amide structure effective in the control of biofilm deposition.

Patent Document 1 teaches the control of biofilm deposition using compounds denoted by the following Formulas (a) or (b). 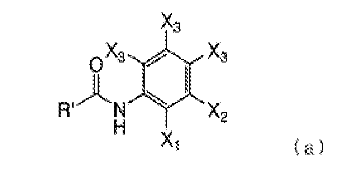 wherein X₁, X₂, and X₃ are independently selected from the group consisting of H, OH, SH, OR¹, SR¹, NH₂, NHR¹, NR¹R², COOR¹, alkyl, cycloalkyl, alkenyl, alkynyl, aryl, heteroaryl, heteroalicyclic, acyl group, carbonyl, cyano, nitro, halogen, and, adjacent X₁, X₂, and X₃ substituents combined, a cycloalkyl of 3 to 8 carbon atoms, a heterocycloalkyl of 3 to 8 members comprising carbon atoms and from 1 to 6 heteroatoms selected from the group consisting of N, S, and O, and an aryl or heteroaryl ring having 3 to 8 members, wherein R¹ and R² are selected from the group consisting of H, alkyl, cycloalkyl, alkenyl, aryl, heteroaryl, carbonyl, and sulfonyl. 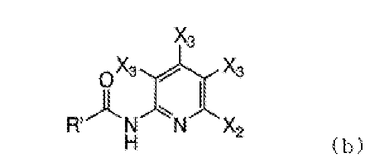 wherein X₂ and X₃ are independently selected from the group consisting of H, OH, SH, OR¹, SR¹, NH₂, NHR¹, NR¹R², COOR¹, alkyl, cycloalkyl, alkenyl, alkynyl, aryl, heteroaryl, heteroalicyclic, acyl group, carbonyl, cyano, nitro, halogen, and, adjacent X₂ and X₃ substituents combined, a cycloalkyl of 3 to 8 carbon atoms, a heterocycloalkyl of 3 to 8 members comprising carbon atoms and from 1 to 6 heteroatoms selected from the group consisting of N, S, and O, and an aryl or heteroaryl ring having 3 to 8 members, wherein R¹ and R² are selected from the group consisting of H, alkyl, cycloalkyl, alkenyl, aryl, heteroaryl, carbonyl, and sulfonyl, and wherein R is selected from the group consisting of CₘH₂ₘ₊₁, wherein m is 1 to 14. 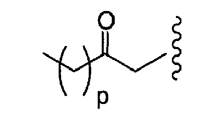 wherein p is 1 to 14, or 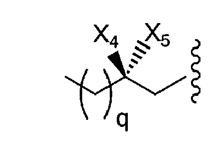 wherein q is 1 to 14, X₄ is OH, NH₂, or SH and X₅ is H, or X₄ is H and X₅ is OH, NH₂, or SH.

Patent Document 1 also teaches that the following compound exhibits autoinducer agonist activity.

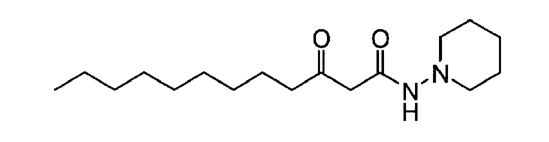

However, regarding those compounds, Patent Document 1 in no way mentions the effect of removing biofilms already formed.
Patent Document 1: Japanese unexamined Patent publication No. 2006-512290

### DISCLOSURE OF INVENTION

As described above, Patent Document 1 does not teach that the disclosed compounds are capable of removing biofilms already formed. However, suppression or inhibition of biofilm deposition may reduce the thickness of the deposited biofilms to some extent, or may even destroy the film if the compounds exhibit desired effect. However, since the microorganisms in the film are still alive, there is the possibility that the microorganisms may form a biofilm again when the effect of the antibacterial agent diminishes or disappears. In view of this, this technology is not an ultimate solution for various biofilm defects such as infections. Therefore, such a drug for inhibiting biofilm deposition is not sufficient to correct the various defects caused by biofilm, or to completely cure biofilm infections. For this reason, there has been demand for a drug that can strip off and remove a deposited biofilm.

An object of the present invention is to provide a biofilm remover to strip off a deposited biofilm. The present invention also provides products containing the biofilm remover, such as an oral composition to prevent or treat intraoral diseases such as periodontitis related to periodontitis pathogenic bacteria. The present invention further provides a compound capable of stripping off a biofilm.

As a result of intensive studies, the inventors of the present invention have found that a compound having certain amide structures is capable of stripping off a deposited biofilm. The inventors also found a new compound in a part of the above-mentioned compound. The following (I) to (III) are included in the scope of the present invention.

### (I) Biofilm Remover (Biofilm Stripping Agent)

I-1. A biofilm remover containing, as an active ingredient, an amide compound denoted by General Formula (1) or salt thereof, 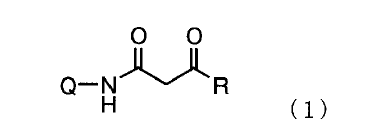 wherein R is a C₁₋₁₁ alkyl group, and Q is a substituent denoted by the following Formulas (Q1), (Q2) or (Q3), 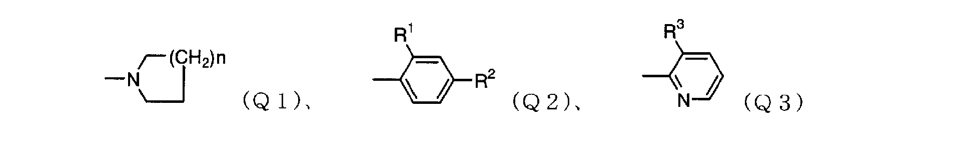 wherein, in Formula (Q1), n is an integer ranging from 0 to 4; in Formula (Q2), R¹ is a C₁₋₄ alkyl group, and R² is a hydroxyl group or carbamoyl group; and in Formula (Q3), R³ is a hydrogen atom, hydroxyl group or carbamoyl group.
1-2. A biofilm remover according to I-1, wherein, in General Formula (1), Q is a substituent denoted by Formula (Q1) wherein n is 1 or 2.
1-3. A biofilm remover according to I-1, wherein, in General Formula (1), R is a C₇₋₁₀ alkyl group, and Q is a substituent denoted by Formula (Q1) wherein n is 1 or 2.
1-4. A biofilm remover according to I-1 or 1-2, wherein, in General Formula (1), R is an n-nonyl group, and Q is a substituent denoted by Formula (Q1) wherein n is 1 or 2. 1-5. A biofilm remover according to I-1, wherein, in General Formula (1), Q is a substituent denoted by Formula (Q2) wherein R¹ and R² are the same as (I-1).
1-6. A biofilm remover according to I-1 or 1-5, wherein, in General Formula (1), Q is a substituent denoted by Formula (Q2) wherein R¹ is a methyl group, and R² is a hydroxyl group or carbamoyl group.
1-7. A biofilm remover according to I-1 or 1-5, wherein, in General Formula (1), R is a C₇₋₁₀ alkyl group, and Q is a substituent denoted by Formula (Q2) wherein R¹ is a methyl group, and R² is a hydroxyl group or carbamoyl group.
1-8. A biofilm remover according to I-1 or 1-5, wherein, in General Formula (1), R is an n-nonyl group, and Q is a substituent denoted by Formula (Q2) wherein R¹ is a methyl group and R² is a hydroxyl group.
1-9. A biofilm remover according to I-1, wherein, in General Formula (1), Q is a substituent denoted by Formula (Q3) wherein R³ is a hydroxyl group.
1-10. A biofilm remover according to I-1 or 1-9, wherein, in General Formula (1), R is a C₁₋₄ alkyl group, and Q is a substituent denoted by Formula (Q3) wherein R³ is a hydroxyl group.
I-11. A biofilm remover according to I-1 or 1-9, wherein, in General Formula (1), R is an n-propyl group, and Q is a substituent denoted by Formula (Q3) wherein R³ is a hydroxyl group. 1-12. A biofilm remover according to any one of I-1 to I-11, wherein the biofilm remover strips off biofilm formed by Pseudomonas aeruginosa.
1-13. A biofilm remover according to any one of I-1 to I-11, wherein the biofilm remover strips off biofilms formed by periodontitis pathogenic bacteria.
1-14. Use of the amide compound denoted by General Formula (1) or salt thereof, as a biofilm remover.
1-15. Use of the amide compound denoted by General Formula (1) or salt thereof, for the preparation of a biofilm remover. 1-16. Use of the amide compound denoted by General Formula (1) or salt thereof, as an active ingredient of a biofilm remover.

### (II) Oral Composition

The biofilm remover according to (1-13) is useful as a component of an oral composition. Therefore, the oral compositions containing the biofilm remover are included in the scope of the present invention.
II-1. An oral composition containing the biofilm remover according to 1-13.
II-2. Use of the biofilm remover according to 1-13, for preparation of an oral composition.
II-3. The use according to II-2, wherein the oral composition is used for the prevention or reduction of oral odor or intraoral diseases caused by periodontitis pathogenic bacteria.
II-4. The biofilm remover according to 1-13, as an active ingredient of an oral composition.
II-5. The biofilm remover according to II-4, wherein the oral composition is used for the prevention or reduction of oral odor or intraoral diseases caused by periodontitis pathogenic bacteria.

### (III) A New Amide Compound or Salt Thereof

New amide compounds and salts thereof having biofilm stripping effect mentioned below are included in the scope of the present invention.
III-1. An amide compound denoted by General Formula (1b) or salt thereof, 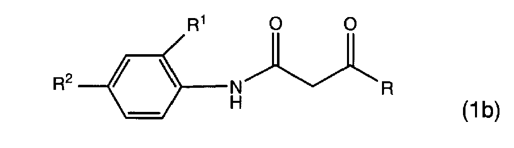 wherein R¹ is a C₁₋₁₁ alkyl group, R² is a C₁₋₄ alkyl group, and R³ is a hydroxyl group or carbamoyl group.
III-2. An amide compound or salt thereof according to III-1, wherein, in General Formula (1b), R¹ is a methyl group.
III-3. An amide compound or salt thereof according to III-1 or
III-2, wherein, in General Formula (1b), R² is a hydroxy group.
III-4. An amide compound or salt thereof according to any one of
III-1 to III-3, wherein, in General Formula (1b), R is a C₇₋₁₀ alkyl group.
III-5. An amide compound or salt thereof according to any one of
III-1 to III-4, wherein, in General Formula (1b), R is an n-nonyl group.
III-6. An amide compound or salt thereof according to any one of
III-1 to III-5, wherein, in General Formula (1b), R is an n-nonyl group, R¹ is a methyl group, and R² is a hydroxy group.
III-7. An amide compound denoted by General Formula (2) or salt thereof, 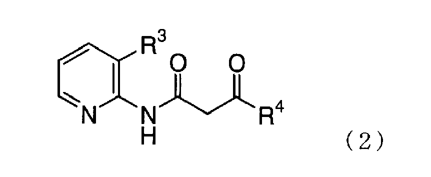 wherein R³ is a hydrogen atom, hydroxyl group or carbamoyl group, and R⁴ is a C₁₋₄ alkyl group.
III-8. An amide compound or salt thereof according to III-7, wherein, in General Formula (2), R³ is a hydroxyl group.
III-9. An amide compound or salt thereof according to III-7 or
III-8, wherein, in General Formula (2), R⁴ is an n-propyl group.
III-10. An amide compound or salt thereof according to III-7, wherein, in General Formula (2), R³ is a hydroxyl group, and R⁴ is an n-propyl group.

### BRIEF DESCRIPTION OF DRAWINGS

FIG. 1 shows a flow cell system. 1: Culture medium bottle (product of Nunc), 2: Pump (4-channel peristaltic pump ISM935: product of ISMATEC), 3: Air removal section (a modified stoppered glass column), 4: Glass cell (Observation Glass Capillary FC91 (1mm×1mm×14mm): product of BioSurface Technology), 5: Waste fluid bottle (product of Nunc), 6: Silicon tube (ϕ1.5mm), 7a, 7b: Three-way turncock (product of Termo), 8a, 8b: Membrane filter (0.44µm: product of Millipore), 9: Culture medium, 10: Waste fluid.
FIG. 2 shows a state of biofilm formation in an experiment (control test) using a control test liquid (3 days, 4 days, 5 days, 6 days, 7 days and 10 days after the addition of the control test liquid).
FIG. 3 shows a comparison result with regard to biofilm exfoliation between an experiment using a test liquid (Compound 1a-1, 100µM) and an experiment using a control test liquid.
FIG. 4 shows a state of biofilm formation in an experiment using a test liquid (Compound 1a-2, 100µM) (3 days, 4 days, 5 days, 6 days, 7 days and 10 days after the addition of the test liquid) .
FIG. 5 shows a state of biofilm formation in an experiment using a test liquid (Compound 1b-1, 100µM) (3 days, 4 days, 5 days, 6 days, 7 days and 10 days after the addition of the test liquid).
FIG. 6 shows a result of biofilm formation in an experiment using a test liquid (Compound 1c-1, 100µM) (3 days, 4 days, 5 days, 6 days, 7 days and 10 days after the addition of the test liquid) .
FIG. 7 shows a state of biofilm formation in an experiment (comparative test) using a test liquid (Comparative Compound 1, 100µM) (3 days, 4 days, 5 days, 6 days, 7 days and 10 days after the addition of the test liquid).

### BEST MODE FOR CARRYING OUT THE INVENTION

### (I) Biofilm Remover

The present invention provides a biofilm remover for stripping off deposited biofilms.

The "biofilm" of the present invention refers to a mucous film-like secretion, formed by microorganisms, that adheres to the surfaces of solids. In the biofilm, plural kinds of coexisting microorganisms form a complex (colony). The "biofilm" may also be described as an aggregation of microorganisms, surrounded by slime-like excrement generated by the microorganisms. The biofilm can adhere to inert solids, or solids such as polymers, plastics, ceramics, metals, glass, or hydroxyapatites that lack a self-governing ability. Additionally, the solid can, of course, be skin, bones, teeth, gingiva, tissues or any other part of living organism.

The biofilm remover of the present invention contains an amide compound denoted by the following Formula (1) or salt thereof, as an active ingredient. 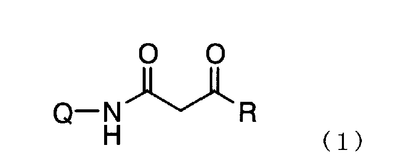 wherein R denotes a C₁₋₁₁ alkyl group, and Q denotes a substituent denoted by the following formulas (Q1), (Q2) or (Q3).

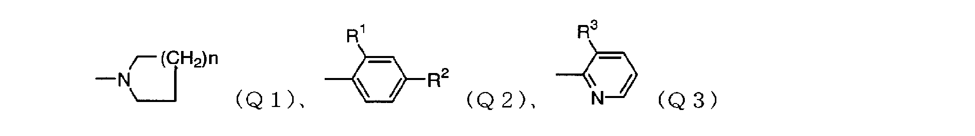

In Formula (Q1), n is an integer ranging from 0 to 4. In formula (Q2), R¹ denotes a C₁₋₄ alkyl group, and R² denotes a hydroxyl group or carbamoyl group. In Formula (Q3), R³ denotes a hydrogen atom, hydroxyl group, or carbamoyl group.

In Formula (1), R denotes a C₁₋₁₁ alkyl group including a straight chain or branched-chain C₁₋₄ alkyl group, such as methyl, ethyl, n-propyl, isopropyl, n-butyl, isobutyl, sec-butyl, or tert-butyl; and a straight chain or branched-chain C₁₋₁₁ alkyl group, such as n-pentyl, 1-methyl-n-butyl, 2-methyl-n-butyl, 3-methyl-n-butyl, 1,1-dimethyl-n-propyl, 1,2-dimethyl-n-propyl, 2,2-dimethyl-n-propyl, 1-ethyl-n-propyl, n-hexyl, 1-methyl-n-pentyl, 2-methyl-n-pentyl, 3-methyl-n-pentyl, 4-methyl-n-pentyl, 1,1-dimethyl-n-butyl, 1,2-dimethyl-n-butyl, 1,3-dimethyl-n-butyl, 2,2-dimethyl-n-butyl, 2,3-dimethyl-n-butyl, 3,3-dimethyl-n-butyl, 1-ethyl-n-butyl, 2-ethyl-n-butyl, 1,1,2-trimethyl-n-propyl, 1,2,2-trimethyl-n-propyl, 1-ethyl-1-methyl-n-propyl, 1-ethyl-2-methyl-n-propyl, 1-methyl-1-ethyl-n-pentyl, n-heptyl, 2-heptyl, 1-ethyl-1,2-dimethyl-n-propyl, 1-ethyl-2,2-dimethyl-n-propyl, n-octyl, 3-octyl, 4-methyl-3-n-heptyl, 6-methyl-2-n-heptyl, 2-propyl-1-n-heptyl, 2,4,4-trimethyl-1-n-pentyl, n-nonyl, 2-nonyl, 2,6-dimethyl-4-n-heptyl, 3-ethyl-2,2-dimethyl-3-n-pentyl, 3,5,5-trimethyl-1-n-hexyl, n-decyl, 2-decyl, 4-decyl, 3,7-dimethyl-1-n-octyl, 3,7-dimethyl-3-n-octyl, n-undecyl, or n-dodecyl.

In the substituent denoted by Formula (Q2) or in the compound denoted by General Formula (2), R¹ or R⁴ denote a C₁₋₄ alkyl group including a straight chain or branched-chain C₁₋₄ alkyl group, such as methyl, ethyl, n-propyl, isopropyl, n-butyl, isobutyl, sec-butyl, or tert-butyl. R¹ is preferably a methyl group or ethyl group, among which a methyl group is particularly preferable. R⁴ is preferably an ethyl group, an n-propyl group, or an n-butyl group, among which an n-propyl group is particularly preferable.

The amide compound denoted by General Formula (1) may also be denoted by the following compounds (1a) to (1c). 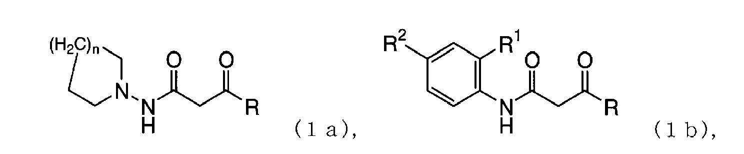 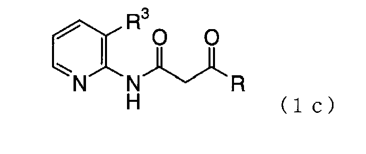 wherein R, R¹, R², R³ and n are the same as those above.

More specifically, among the amino compounds defined by General Formula (1), the amide compound (1a) corresponds to the amide compound with a substituent Q denoted by Formula (Q1), and amide compounds (1b) and (1c) respectively correspond to the amino compound with a substituent Q denoted by Formula (Q2) and the amino compound with a substituent Q denoted by Formula (Q3).

Note that, among these amide compounds, the amide compound (1b) is a new compound not disclosed in Patent Document 1.

The amide compound denoted by Formula (2) is also a new compound not disclosed in Patent Document 1. 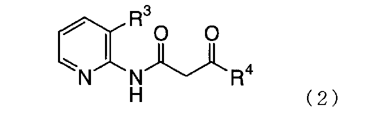 wherein R³ denotes a hydrogen atom, hydroxyl group or carbamoyl group, and R⁴ denotes a C₁₋₄ alkyl group.

Among the amide compounds defined by Formula (1a), a compound in which n = 1 or 2 is preferable, and a compound in which n = 1 is more preferable. In the amide compound (1a), R is preferably a C₁₋₁₁ alkyl group, more preferably a C₁₋₄ alkyl group or a C₇₋₁₀ alkyl group, further preferably a C₇₋₁₀ alkyl group, and particularly preferably an n-nonyl group. Note that the compound (1a), in which n = 1 and R is an n-nonyl group, was previously disclosed in Patent Document 1.

Among the amide compounds defined by Formula (1b), a compound in which R¹ is a C₁₋₂ straight chain alkyl group such as a methyl group or an ethyl group is preferable. A compound in which R¹ is a methyl group is more preferable. It is further preferable in the amide compound defined by Formula (1b) that R¹ is a methyl group and R² is a hydroxyl group. Further, R is preferably a C₁₋₄ alkyl group or a C₇₋₁₀ alkyl group, more preferably a C₇₋₁₀ alkyl group, particularly preferably an n-nonyl group.

Among the amide compounds defined by Formula (1c), a compound in which R³ is a hydroxyl group is preferable. It is further preferable in the amide compound (1c) that R is a C₁₋₁₁ alkyl group, more preferably a C₁₋₄ alkyl group. This compound corresponds to the compound (2). Further, the compound (1c), in which R is an n-nonyl group, was previously disclosed in Patent Document 1. For the compound (2), it is preferable that R³ be a hydroxyl group and R an n-propyl group.

The amide compound denoted by Formula (1) of the present invention forms salt with an acid. The acid which forms salt with the amide compound (1) is not particularly limited, but an acid for pharmaceutical use is preferred. Examples of the acid include inorganic acids such as hydrochloric acid, nitric acid, sulfuric acid, or phosphoric acid; and organic acids such as formic acid, acetic acid, tartaric acid, maleic acid, malic acid, citric acid, salicylic acid, benzoic acid or ascorbic acid.

The amide compound (1) used for the present invention can be manufactured by a method denoted by Reaction Formula -1, for example. 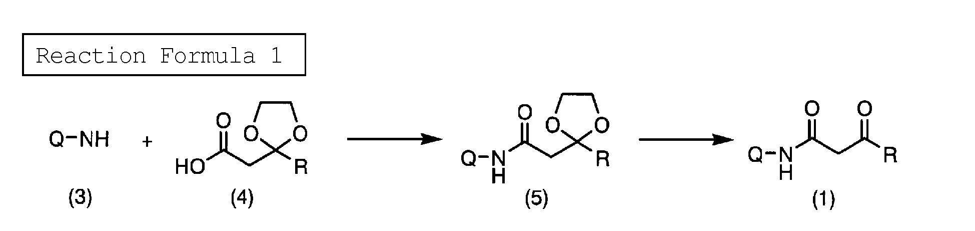 wherein Q and R are the same as those above.

According to Reaction Formula 1, the amine compound of Formula (3) and the carboxylic acid compound of Formula (4) are reacted to become the amide compound of Formula (5), and then induced the amide compound of Formula (1).

The amide compound (5) can be manufactured by reacting the amine compound (3) and the carboxylic acid (4) in an inactive solvent in the presence of an appropriate condensing agent.

Examples of the condensing agent include an acid halide-forming agent such as phosphorus trichloride, phosphorus tribromide, phosphorus pentachloride, phosphorus oxychloride, thionyl chloride; a mixed acid anhydride-forming agent such as ethyl chloroformate, or chlorinated methane sulfonyl; carbodiimides such as N,N'-dicyclohexyl carbodiimide(DCC), diisopropyl carbodiimide, or 1-ethyl-3- dimethylaminopropyl carbodiimide. Other condensing agents, such as N'N-carbonyldiimidazole, 2-ethoxy-N-ethoxycarbonyl-1,2-dihydroquinoline (EEDQ), or triphenylphosphine-carbon tetrachloride (complex), may also be used.

Examples of the inactive solvent include aromatic hydrocarbons such as benzene, toluene, or xylene; halogenated aromatic hydrocarbons such as chlorobenzene, or dichlorobenzene; aliphatic hydrocarbons such as hexane, cyclohexane, or petroleum ether; halogenated aliphatic hydrocarbons such as dichloromethane, 1,2-chloroethane, chloroform, or carbon tetrachloride; ethers such as diethyl ether, diisopropyl ether, dioxane, tetrahydrofuran, ethyleneglycoldimethylether, or ethyleneglycoldiethylether; ketones such as acetone, 2-butanone, or methylisobutylketone; nitriles such as acetonitrile, propionitrile, or benzonitrile; amides such as N,N-dimethylformamide, or hexamethylphosphoric triamide (HMPA); sulfoxides such as dimethylsulfoxide; and mixtures of those.

The reaction can be induced by using 0.8 to 5 mol, more preferably, 1 to 3 mol of the amine compound (3), per mol of the carboxylic acid compound (4). The amount of the condensing agent is not limited, but typically 0.8 to 5 mol, more preferably 1 to 3 mol, per mol of the carboxylic acid compound (4).

The reaction temperature is not particularly limited, but typically set in a range from -10°C up to the boiling point of the solvent. The reaction time varies depending on the proportion of the carboxylic acid compound (4) to the amine compound (3), the concentration of the condensing agent, or the reaction temperature, but is typically controlled between 5 to 10 hours.

Note that the carboxylic acid compound (4) used in the reaction above corresponds to the compound in which the 3-carbonyl group of the 3-oxocarboxylic acid of Formula (6) is protected by ethylene glycol. 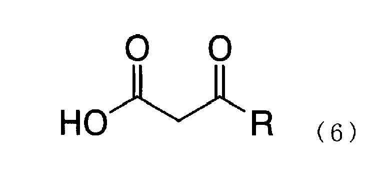 In the formula, R is the same as above.

In the reaction above, the protecting group of the 3-carbonyl group of the carboxylic acid compound (4) is not limited to ethylene glycol, and may be any arbitrary protecting group. One example of the protecting group may be the protecting group of a carbonyl group disclosed in "Protective Groups in Organic Synthesis, Theodora W. Greene, 1981". More specifically, the reaction above may be performed by using a carboxylic acid compound with a 3-carbonyl group protected by a protecting group, instead of the carboxylic acid compound (4).

The amide compound (5) thus obtained was then subjected to deprotection to produce the target amide compound (1), a process which can be done using any known method depending on the type of the protecting group.

Note that the amine compound (3) and carboxylic acid compound (4) used as the raw materials of the reaction are commercially available. Easy manufacturing methods for those compounds have also been publicly disclosed.

The target amide compound (1) thus obtained can be easily refined by isolating it from the reaction mixture via a general isolation method such as column chromatography or recrystallization.

The biofilm remover according to the present invention may have any form as long as it contains the amide compound (1) or salt thereof as an active ingredient. For example, the biofilm remover may be the amide compound (1) or the salt thereof itself, or a composition made by mixing an arbitrary carrier or additive with the amide compound (1) or a salt thereof and processing the mixture into a desired form with a known method according to the usage. Though it is not particularly limited, possible forms of the biofilm remover according to the present invention include solid pruducts such as tablets, powders, granules, pills, powder syrups or capsules (hard capsules, soft capsules); paste or gel products such as creams, ointments or gels; liquid products such as solutions, suspensions or emulsions syrups, elixirs, sprays or aerosols.

Insofar as the resulting biofilm remover ensures sufficient biofilm removal, the content of the amide compound (1) or salt thereof in the biofilm remover of the present invention is not limited. In other words, to ensure the desired biofilm removal effect, the content of the amide compound (1) or salt thereof should be adjusted in a range from 0.001 to 99 wt%, preferably 0.01 to 50 wt% or 0.05 to 10 wt%, per 100wt% of biofilm remover.

The biofilm remover of the present invention thus contains the amide compound (1) or salt thereof at a ratio to ensure the biofilm removal effect, and may contain other components according to the usage or purpose of the biofilm remover insofar as the biofilm removal effect is not impaired. Though it is not particularly limited, possible examples of the other components include general carriers for drug formulation such as diluent bases, binders, dispersants, viscosity improvers, lubricants, pH adjusters, solubilizing agents; and other agents such as antibiotics, antimicrobial agents, disinfectants, antiseptics, builders, bleaches, enzymes, chelating agents, antifoaming agents, colorants (dyes, pigments, etc.), softening agents, moisturizers, surfactants, antioxidants, perfumes, corrective substances, odor-masking agents and solvents.

In addition to the amide compound (1) or salt thereof, the biofilm remover of the present invention may contain an antimicrobial agent or a disinfectant, for example, a tetracycline disinfectant such as minocycline hydrochloride; cation disinfectants such as triclosan, cetylpyridinium chloride, or benzethonium chloride; or macrolide antibiotic. The biofilm remover of the present invention may also contain compounds for improving the activity of the antimicrobial agent or disinfectant. Examples of the compound include basic amino acids such as arginine, lysine, or histidine; various enzymes including starch modification enzymes such as farnesol, transglucosidase, or CGTase; and starch hydrolases such as α-amylase.

The biofilm remover of the present invention is applicable anywhere a biofilm has developed to detrimental effect. The biofilm remover of the present invention exhibits an excellent biofilm removal effect, particularly on biofilms formed by Pseudomonas aeruginosa or periodontitis pathogenic bacteria. Examples of periodontitis pathogenic bacterias include Porphyromonas gingivalis, Tannerella forsythensis, Actinobacillusactinomycetemcomitans, Prevotella intermedia, Eikenellacorrodens, Campylobacter rectus, Fusobacterium necleatum, Treponemadenticola, Actinomyces naeslundii, and Streptococcus mutans.

The biofilm remover of the present invention can be used, for example, in the following manner to remove biofilms deposited in industrial areas, circulating-type bathtubs, or the like. The biofilm stripping agent of the present invention, having been processed into a suspension liquid, water-dispersible powder, or water-soluble powder, is circulated in the pipes of the target equipment, or sprayed on the target portion of the equipment. The biofilm remover of the present invention can also take the form of a high-concentration liquid, or a solid preparation such as tablet, powder, or grain agent supplied to a water tank so that the remover diluted or dissolved in the water is applied to the target portion. Further, the biofilm remover of the present invention can be processed into medicinal preparations suitable for oral administration, parenteral administration, or local administration. Moreover, as described later, the biofilm remover of the present invention can be processed into various oral-sanitation products such as toothbrushing agents, mouth deodorants, mouth washes, gingival medicines, gums, gargles, artificial teeth, or cleaning agents for dental materials.

The appropriate usage quantity of the biofilm remover of the present invention varies depending on the target object or dosage form (it differs particularly for sustained-release formulation), and therefore cannot be clearly defined. However, when used to prevent or treat biofilm infection, for example, an appropriate per-day dosage amount of the biofilm remover of the present invention is typically 1 ng/mL to 100mg/mL, preferably 10ng/mL to 10mg/mL, in the absolute quantity, typically 1ng to 500mg, on the basis of the dosage of the amide compound (1) or salt thereof of the present invention (e.g., the gross quantity for humans is 300mg).

### (II) Oral Composition

The present invention provides an oral composition containing the biofilm remover. The present invention was made based on the fact that the biofilm remover of the present invention having the amide compound (1) or salt thereof as an active ingredient has a particularly notable effect of stripping off a biofilm formed by periodontitis pathogenic bacteria.

The oral composition of the present invention is typically a composition dedicatedly used for oral treatments to prevent or treat oral diseases such as periodontitis related to pathogenic bacteria. Examples of such a composition include toothbrushing agents such as tooth pastes, toothbrushing powders, toothbrushing liquids, or infiltrative toothbrushing agents; mouth deodorants and mouth washes in the form of troches, tablets, liquids, gums, oleasters or films; and gingival medicines in the form of creams, ointments, or gels. The oral composition according to the present invention may also be a composition used dedicatedly for the care of medical oral materials, such as artificial teeth or other dental materials, to prevent oral diseases. Examples of such compositions include cleaning agents for artificial teeth or dental materials.

The proportion of the biofilm remover in the oral composition is preferably adjusted so that the content (gross amount) of the amide compound (1) or salt thereof, that is an active ingredient of the biofilm remover, falls within a range from 0.001 to 99 wt%, preferably 0.01 to 50 wt%, more preferably 0.05 to 10 wt%, per 100wt% of the composition.

According to the type or form of the composition, as needed, the oral composition of the present invention may contain the following components in a general usage range, in addition to the foregoing components.

### Abrasive

Silica abrasives such as silica gel, precipitable silica, igneous silica, hydrous silicic acid, anhydrous silicic acid, titanium silicate, zeolite, aluminosilicate, and zirconosilicate; monobasic calcium phosphate, dibasic calcium phosphate dihydrate, dibasic calcium phosphate non-hydrates, calcium pyrophosphate, tribasic magnesium phosphate, tribasic calcium phosphate, aluminum hydroxide, alumina, calcium carbonate light, calcium carbonate heavy, magnesium carbonate, tribasic magnesium phosphate, zirconium silicate, insoluble sodium metaphosphate, insoluble calcium metaphosphate, titanium oxide, and synthetic resin abrasive. These abrasives may be used solely or in combination. When incorporating such abrasives (e.g., in dentifrices), the amount is not particularly limited, but preferably 3 to 80 wt%, and more preferably 10 to 50 wt%, per 100wt% of oral composition.

### Humectant and Viscous Agent

Polyhydric alcohols such as glycerin, concentrated glycerin, diglycerin, ethylene glycol, dipropylene glycol, polyethylene glycol, propylene glycol, polypropylene glycol, or 1,3-butyleneglycol; and sugar-alcohols such as xylitol, maltitol, or lactol. These humectants and viscous agents may be used solely or in combination.

### Binder

Alginates and derivatives thereof such as alginic acid, sodium alginate, propylene glycol alginate ester, calcium-containing sodium alginate, potassium alginate, calcium alginate, ammonium alginate; gums such as carrageenan (mainly Iota, L; Lambda, λ; and Kappa, k), xanthan gum, tragacanth, karaya gum, gum arabic, locust bean gum, or guar gum; celluloses such as carboxymethylcellulose sodium, methylcellulose, ethylcellulose, cellulose acetate, or hydroxyethylcellulose sodium; gelatin, agar, polyvinyl alcohol, sodium polyacrylate, carboxyvinyl polymer, polyvinyl pyrrolidone, carbopol, silica gel, aluminium silica gel, and thickening nature silica. These binders may be used solely or in combination. When incorporating such binders (e.g., in dentifrices), the amount is not particularly limited, but preferably 0.1 to 10 wt%, per 100wt% of oral composition.

### Foaming Agent

Sodium lauryl sulfate, lauroylsarcosine sodium, alkyl sulfo monosodium succinate, palm-oil-fatty-acid mono-glycerol sulfone sodium, α-olefin sulfone sodium, N-acylamino acid salt such as N-acylglutamate, 2-alkyl-N-carboxymethyl-N-hydroxyethyl imidazolinium betaine, maltitol fatty acid ester, sucrose fatty acid ester, polyglyceryl fatty acid ester, fatty acid diethanolamide, polyoxyethylenesorbitan monostearate, polyoxyethylene hydrogenated castor oil, or polyoxyethylene fatty acid ester. These foaming agents may be used solely or in combination.

### Surfactant

As the surfactant, anion surfactants, cation surfactants, nonionic surfactants, and amphoteric surfactants are all applicable.

Examples of anion surfactants include sodium lauryl sulfate, sodium myristyl sulfate, N-lauroylsarcosinic acid sodium salt, N-myristoylsarcosinic acid sodium salt, sodium dodecylbenzenesulfonate, hydrogenation coconut fatty acid monoglyceride mono-sodium sulfate, sodium lauryl sulfosulfate, α-olefin sulfonate sodium, N-acylglutamates such as N-palmitoyl sodium glutamate, and N-acyltaurates such as N-methyl-N-acyltaurine sodium. Examples of nonionic surfactants include sucrose fatty acid esters such as sucrose fatty acid ester or maltose fatty acid ester, sugar-alcohol fatty acid esters such as maltitol fatty acid ester and lactol fatty acid ester, alkylol amide, polyoxyethylene sorbitan fatty acid esters such as polyoxyethylenesorbitan monostearate, polyoxyethylene fatty acid esters such as polyoxyethylene hydrogenated castor oil, fatty acid ethanolamides such as lauryl acid mono- or di- ethanolamide, sorbitan fatty acid ester, polyoxyethylene higher alcohol ether, polyoxyethylene polyoxypropylene copolymer, polyoxyethylene polyoxypropylene fatty acid ester, polyglyceryl fatty acid ester, and pluronic. Examples of amphoteric surfactants include 2-alkyl-N-carboxymethyl-N-hydroxyethyl imidazolium betaine, N-alkyldiaminoethyl glycine such as N-lauryl diaminoethyl glycine or N-myristyl diaminoethyl glycine, and N-alkyl-1-hydroxyethylimidazolin betaine sodium. These surfactants may be used solely or in combination.

### Sweetening Agent

Saccharin sodium, aspartame, stevioside, stevia extract, paramethoxy cinnamic aldehyde, neohesperidyl dihydrochalcone, perillartine, glycyrrhizine, and thaumatin. These sweetening agents may be used solely or in combination.

### Antiseptic

Parabens such as methylparaben, ethylparaben, propylparaben, or butylparaben; sodium benzoate, phenoxyethanol, and alkyldiaminoethylglycine hydrochloride. These antiseptics may be used solely or in combination.

### Aromatic Component

1-menthol, anethole, menthone, cineole, limonene, carvone, methyl salicylate, ethyl butyrate, eugenol, thymol, n-decylalcohol, citronellol, α-terpineol, citronellyl acetate, linalool, ethyl linalool, vanillin, peppermint, cinnamic aldehyde, and trans-2-hexenal. These aromatic components may be used solely or in combination. Note that, these components may be purified products, or may be crude products of essential oils containing these components (for example, lemon oil, orange oil, sage oil, rosemary oil, cassia and cinnamon oil, pimento oil, cinnamon leaf oil, beefsteak plant oil, wintergreen oil, oil of cloves, or eucalyptus oil).

Further, in addition to the foregoing aromatic components, other components or essential oil such as aliphatic alcohol or ester thereof, terpene carbon hydride, phenolether, aldehyde, ketone, or lactone may be used insofar as the effects of the present invention are not impaired. The amount of these aromatic components is preferably 0.02 to 2 wt%, per 100 wt% of the whole oral composition.

### Antimicrobial Component

Antibacterial metal, such as silver, copper, zinc, or metal salts thereof with low water solubility (e.g., silver oxide, silver chloride, silver carbonate, silver phosphate, copper hydroxide, copper gluconate, zinc oxide, zinc citrate, zinc stearate, and zinc trichlorophenate, zinc hydroxide, zinc oxalate, zinc phosphate), copper chlorophyll, cetylpyridium chloride, benzalkonium chloride, triclosan, hinoki thiol, lysozyme chloride.

### Antiseptic Component

Nonionic antimicrobial agents such as parabens, sodium benzoate or triclosan; cationic antiseptic agents such as benzethonium chloride or cetylpyridinium chloride.

### Oral Active Ingredient

Lysozyme chloride, sodium fluoride, potassium fluoride, sodium monofluorophosphate, polyethyleneglycol, polyvinyl pyrrolidone, hinoki thiol, ascorbic acid, ascorbic acid salts, chlorhexidine salts, cetylpyridinium chloride, benzalkonium chloride, benzethonium chloride, bisabolol, triclosan, isopropylmethylphenol, tocopherol acetate, epsilon(ε)-aminocaproic acid, tranexamic acid, aluminium hydroxyl allantoin, aluminum lactate, dihydrocholesterol, glycyrrhetinic acid, glycyrrhizinates, copper chlorophyllin salt, sodium chloride, guaiazulene sulfonate, dextranase, pyridoxine hydrochloride, tranexamic acid, sodium chloride, Vitamins C and E, various enzymes (e.g., dextranase, amylase, protease, mutanase, or pectinase), tartar control agents such as azulene or polyphosphate, nicotine stripping agents such as polyethylene glycol or polyvinyl pyrrolidone, and hyperesthesia prophylactic agents such as aluminum lactate or potassium nitrate. These oral active ingredients may be used solely or in combination.

### Other Additives

Pigments such as Food Blue No. 1, titanium oxide, antioxidants such as dibutylhydroxytoluene, and flavoring substances such as tea leaf dry distilled solution or sodium glutamate.

The oral composition of the present invention can be manufactured by any common procedure. The production of the oral composition in the form of, for example, toothpaste, is completed by being packed in an aluminium tube, laminated tube, glass evaporation tube, plastic tube, plastic bottle, aerosol container or the like before being marketed to allow the consumer to apply it to the target portion.

The oral composition of the present invention removes intraoral biofilms formed by periodontitis pathogenic bacteria, thereby effectively preventing generation of bacterial flora. Further, by incorporating an antimicrobial agent in the remover, the improved antimicrobial effect will further increase the quality of the oral composition. Therefore, the oral composition of the present invention is effective for the prevention or treatment of periodontosis and periodontal diseases (for example, gingivitis). Further, the oral composition of the present invention is effective for prevention or removal of oral odor due to periodontosis or periodontal diseases (for example, gingivitis) .

### EXAMPLES

The following describes production examples and experiment examples of the amide compound (1) according to the present invention to more specifically explain the present invention. The present invention is, however, not limited to those examples.

### Reference Example 1

### Synthesis of 3,3- ethylene glycosyldodecanoyl acid

### (1) Synthesis of 3-oxododecanoyl acid ethyl ester

7.20g (50mmol) of Meldrum's acid and 5.56g (55mmol) of triethylamine were dissolved in 50mL of dichloromethane. 10.48g (55mmol) of decanoyl chloride was dropped in this solution while cooling the solution with ice. The reaction liquid was stirred for 12 hours at room temperature, and was concentrated under reduced pressure. The resulting residue was subjected to extraction with ethyl acetate. The obtained ethyl acetate layer was washed with diluted hydrochloric acid and saturated saline, dried with magnesium sulfate, and concentrated under reduced pressure. The resulting residue was purified by silica gel chromatography (developing solvent: hexane/ethyl acetate=3/7) to obtain a Meldrum's acid derivative.

The obtained Meldrum's acid derivative was circulated in ethanol for two hours, and was concentrated under reduced pressure. The resulting residue was purified by silica gel chromatography (developing solvent: hexane/ethyl acetate=3/7). As a result, 3-oxododecanoyl acid ethyl ester denoted by the following formula was obtained. 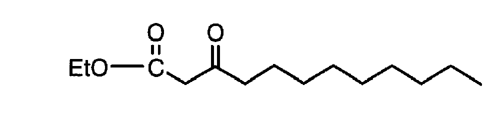 Quantity Yield: 11.85g (0.69mmol)
Percent Yield: 99%
1H-NMR (CDCl₃, 500MHz); 0.91ppm (t, 3H), 1.30ppm (m, 15H), 1.62ppm (m, 2H), 2.55ppm(m, 2H), 3.45ppm (s, 2H), 4.20ppm (q, 2H).

### (2) Synthesis of 3,3-ethyleneglycosyl dodecanoyl acid ethyl ester

6.05g (25mmol) of the 3-oxododecanoyl acid ethyl ester obtained in the process (1), 7.75g (125mmol) of ethylene glycol, and 0.48g (2.5mmol) of p-toluene sulfonic acid monohydrate were dissolved in 50mL of benzene, and the solution was circulated for six hours by a circulation device incorporating the Dean and Stark device. After cooling, the reaction liquid was washed with saturated sodium bicarbonate water, dried with magnesium sulfate, and concentrated under reduced pressure. The resulting residue was purified by silica gel chromatography (developing solvent: hexane/ethyl acetate=5/5). As a result, 3,3-ethylene glycosyl dodecanoyl acid ethyl ester denoted by the following formula was obtained. 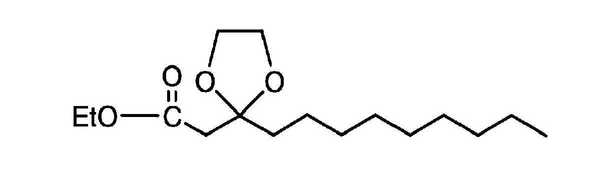 Quantity Yield: 6.99g(24.5mmol)
Percent Yield: 98%
¹H-NMR (CDCl₃, 500MHz); 0.91ppm (t, 3H), 1.29ppm (m, 15H), 1.33ppm (m, 2H), 1.81ppm (m, 2H), 2.67ppm (s, 2H), 4.01ppm (m, 4H), 4.19ppm (q, 2H).

### (3) Synthesis of 3,3-ethyleneglycosyl dodecanoyl acid

5.72g (20mmol) of 3,3-ethyleneglycosyl dodecanoyl acid ethyl ester obtained in the process (2), 4.20g (100mmol) of lithium hydroxide monohydrate were dissolved in a mixed solution of 50mL of tetrahydrofuran and 50mL of distilled water, and the solution was stirred at room temperature for 12 hours. The reaction liquid was neutralized by diluted hydrochloric acid, and was concentrated under reduced pressure. The residue was subjected to extraction with ethyl acetate. The obtained organic layer was washed with saturated saline. The layer was dried with magnesium sulfate, and concentrated under reduced pressure. The resulting residue was purified by silica gel chromatography (developing solvent: ethyl acetate/methanol=3/7) to obtain 3,3-ethylene glycosyldodecanoyl acid. 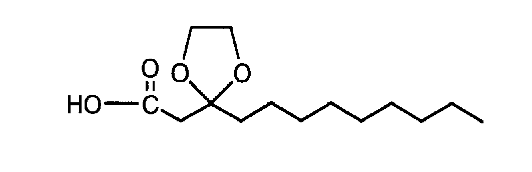 Quantity Yield: 3.71g (14.4mmol)
Percent Yield: 72%
¹H-NMR (CDCl₃, 500MHz); 0.84ppm (t, 3H), 1.22ppm (m, 12H), 1.33ppm (m, 2H), 1.76ppm (m, 2H), 2.65ppm (s, 2H), 3.97ppm (m, 4H).

### Reference Example 2

### Synthesis of 3,3-ethylene glycosylhexanoyl acid

### (1) Synthesis of 3-oxohexanoyl acid ethyl ester

6.15g (42.7mmol) of Meldrum's acid and 8.63g (85.4mmol) of triethylamine were dissolved in 75mL of dichloromethane. 5.00g (46.9mmol) of butylic chloride was dropped in the solution while cooling the solution with ice. The reaction liquid was stirred for 12 hours at room temperature, and was concentrated under reduced pressure. The residue was subjected to extraction with ethyl acetate. The obtained organic layer was washed with diluted hydrochloric acid and saturated saline. The layer was dried with magnesium sulfate, and concentrated under reduced pressure. The resulting residue was purified by silica gel chromatography (developing solvent: hexane/ethyl acetate=8/2) to obtain a Meldrum's acid derivative.

The obtained Meldrum's acid derivative was circulated in ethanol for two hours, and was concentrated under reduced pressure. The resulting residue was purified by silica gel chromatography (developing solvent: hexane/ethyl acetate=8/2). As a result, 3-oxo hexanoyl acid ethyl ester denoted by the following formula was obtained. 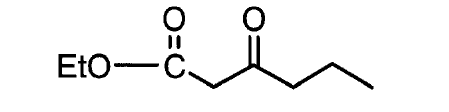 Quantity Yield: 4.85g (30.7mmol)
Percent Yield: 72%
¹H-NMR (CDCl₃, 500MHz); 0.94ppm (t, 3H), 1.30ppm (t, 3H), 1.66ppm (m, 2H), 2.54ppm (m, 2H), 3.45ppm (s, 2H), 4.22ppm (q, 2H).

### (2) Synthesis of 3,3-ethyleneglycosyl hexanoyl acid ethyl ester

540g (3.42mmol) of the 3-oxohexanoyl acid ethyl ester obtained in the process (1), 2.12g (34.2mmol) of ethylene glycol, and 65mg (0.4mmol) of p-toluene sulfonic acid monohydrate were dissolved in 50mL of benzene, and the solution was circulated for six hours by a circulation device incorporating the Dean and Stark device. After cooling, the reaction liquid was washed with saturated sodium bicarbonate water, dried with magnesium sulfate, and concentrated under reduced pressure. The resulting residue was purified by silica gel chromatography (developing solvent: hexane/ethyl acetate=6/4). As a result, 3,3-ethyleneglycosyl hexanoyl acid ethyl ester denoted by the following formula was obtained. 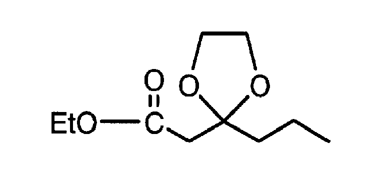 Quantity Yield: 620mg (3.1mmol)
Percent Yield: 90%
¹H-NMR (CDCl₃, 500MHz); 0.94ppm (t, 3H), 1.29ppm (t, 3H), 1.45ppm (m, 2H), 1.81ppm (m, 2H), 2.67ppm (s, 2H), 4.00ppm (m, 4H), 4.20ppm (q, 2H).

### (3)Synthesis of 3,3-ethyleneglycosyl hexanoyl acid

620mg (3.1mmol) of 3,3-ethyleneglycosyl hexanoyl acid ethyl ester obtained in the process (2), 718mg (17.1mmol) of lithium hydroxide monohydrate were dissolved in a mixed solution of 25mL of tetrahydrofuran and 25mL of distilled water, and the solution was stirred at room temperature for 12 hours. The reaction liquid was neutralized by diluted hydrochloric acid, and was concentrated under reduced pressure. The residue was subjected to extraction with ethyl acetate. The obtained organic layer was washed with saturated saline, dried with magnesium sulfate, and concentrated under reduced pressure. The resulting residue was purified by silica gel chromatography (developing solvent: ethyl acetate/methanol=2/8) to obtain 3,3-ethyleneglycosyl hexanoyl acid denoted by the following formula. 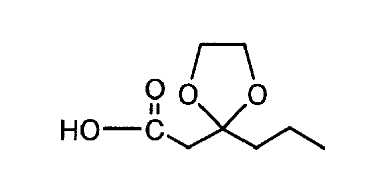 Quantity Yield: 331mg(1.9mmol)
Percent Yield: 62%
¹H-NMR (CDCl₃, 500MHz); 0.69ppm (t, 3H), 1.17ppm (m, 2H), 1.55ppm (m, 2H), 2.32ppm (s, 2H), 3.80ppm (m, 4H).

### Production Example 1

### Production of N-(pyrrolidin-1-yl)-3-oxododecanoyl amide (compound 1a-1)

### (1) Synthesis of N-(pyrrolidin-1-yl)-3,3-ethyleneglycosyl dodecanoyl amide

0.28g (1.1mmol) of 3,3-ethylene glycosyldodecanoyl acid obtained in Reference Example 1 and 0.09g (1mmol) of 1-aminopyrrolidine was dissolved in 10mL of dichloromethane. 0.13g (1.1mmol) of dimethylaminopyridine, 0.15g (1.2mmol) of diisopropylethylamine and 0.21g (1.1mmol) of 1-ethyl-3-dimethylaminopropyl carbodiimide was added to the solution, and the mixture was stirred at room temperature for 12 hours. The reaction liquid was concentrated under reduced pressure, and the residue was subjected to extraction with ethyl acetate. The obtained organic layer was washed with diluted hydrochloric acid and saturated saline, dried with magnesium sulfate, and concentrated under reduced pressure. The resulting residue was purified by silica gel chromatography (developing solvent: ethyl acetate/methanol=4/6) to obtain N-(pyrrolidin-1-yl)-3,3-ethyleneglycosyl dodecanoyl amide denoted by the following formula. 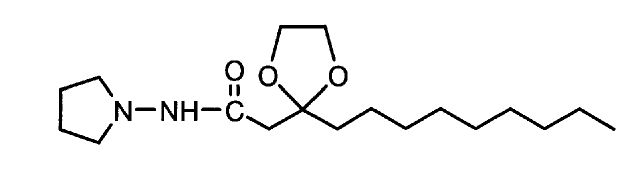 Quantity Yield: 0.24g(0.74mmol)
Percent Yield: 68%
¹H-NMR (CDCl₃, 500MHz); 0.88ppm (t, 3H), 1.23ppm (m, 12H), 1.38ppm (m, 2H), 1.67ppm (m, 2H), 1.82ppm (m, 2H), 1.87ppm (m, 2H), 2.88ppm (m, 4H), 3.48ppm (s, 2H), 3.99ppm (m, 4H), 7.04ppm (br, 1H) .

### (2) Synthesis of N-(pyrrolidin-1-yl)-3-oxododecanoyl amide

5mL of trifluoroacetic acid was added to 0.23g (0.70mmol) of the N-(pyrrolidin-1-yl)-3,3-ethyleneglycosyldodecanoyl amide obtained in the foregoing process, and the mixture was stirred at room temperature for 18 hours. The reaction liquid was neutralized by 5% sodium hydroxide aqueous solution, and the residue was subjected to extraction with ethyl acetate. The obtained organic layer was washed well with saturated sodium bicarbonate water, dried with magnesium sulfate, and concentrated under reduced pressure. The resulting residue was purified by silica gel chromatography (developing solvent: ethyl acetate/methanol=4/6) to obtain N-(pyrrolidin-1-yl)-3-oxododecanoyl amide (Compound 1a-1). The compound 1a-1 corresponds to the amide compound defined by Formula (1a) in which n = 1, and R is an n-nonyl group. 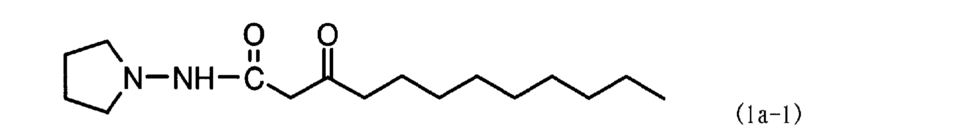 Quantity Yield: 0.18g(0.64mmol)
Percent Yield: 92%
¹H-NMR (CDCl₃, 500MHz); 0.90ppm (t, 3H), 1.28ppm (m, 13H), 1.62ppm (m, 3H), 1.86ppm (m, 2H), 1.91ppm (m, 2H), 2.54ppm (q, 2H), 2.92ppm (m, 2H), 3.46ppm (s, 0.7H), 3.59ppm (s, 1.3H), 3.46ppm(br, 0.7H), 3.59ppm (br, 0.3H).

### Production Example 2

### Production of N-(piperidine-1-yl)-3-oxododecanoyl amide (Compound 1a-2)

### (1) Synthesis of N-(piperidine-1-yl)-3,3-ethyleneglycosyldodecanoyl amide

0.28g (1.1mmol) of 3,3-ethylene glycosyldodecanoyl acid obtained in Reference Example 1 and 0.10g (1mmol) of 1-aminopiperidine were dissolved in 10mL of dichloromethane. 0.13g (1.1mmol) of dimethylaminopyridine, 0.15g (1.2mmol) of diisopropylethylamine, and 0.21g (1.1mmol) of 1-ethyl-3-dimethylaminopropyl carbodiimide were added to the solution, and the mixture was stirred at room temperature for 12 hours. The reaction liquid was concentrated under reduced pressure, and the residue was subjected to extraction with ethyl acetate. The obtained organic layer was washed with diluted hydrochloric acid and saturated saline, dried with magnesium sulfate, and concentrated under reduced pressure. The resulting residue was purified by silica gel chromatography (developing solvent: ethyl acetate/methanol=4/6) to obtain N-(piperidine-1-yl)-3,3-ethyleneglycosyldodecanoyl amide denoted by the following formula. 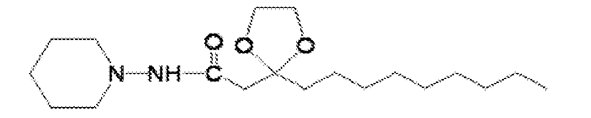 Quantity Yield: 0.23g (0.68mmol)
Percent Yield: 68%
¹H-NMR (CDCl₃, 500MHz); 0.88ppm (t, 3H), 1.28ppm (m, 12H), 1.55ppm (m, 6H), 1.75ppm (m, 2H), 2.48ppm (dm, d=400Hz, 2H), 2.56ppm (m, 2H), 2.67ppm (dm, d=400Hz, 2H), 3.48ppm (s, 2H), 4.01ppm (m, 4H), 7.08ppm (br, 1H).

### (2) Synthesis of N-(piperidine-1-yl)-3-oxododecanoyl amide

5mL of trifluoroacetic acid was added to 0.23g (0.68mmol) of the N-(piperidine-1-yl)-3,3-ethyleneglycosyldodecanoyl amide obtained in the foregoing process, and the mixture was stirred at room temperature for 18 hours. The reaction liquid was neutralized by 5% sodium hydroxide aqueous solution, and the residue was subjected to extraction with ethyl acetate. The obtained organic layer was washed well with saturated sodium bicarbonate water, dried with magnesium sulfate, and concentrated under reduced pressure. The resulting residue was purified by silica gel chromatography (developing solvent: ethyl acetate/methanol=4/6) to obtain N-(piperidine-1-yl)-3-oxododecanoyl amide (Compound 1a-2). The compound 1a-2 corresponds to the amide compound defined by Formula (1a) in which n = 2, and R is an n-nonyl group. 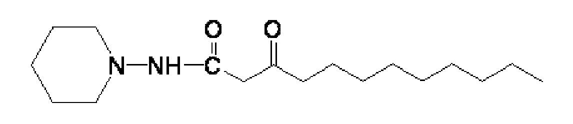 Quantity Yield: 0.17g (0.58mmol)
Percent Yield: 85%
¹H-NMR (CDCl₃, 500MHz); 0.88ppm (t, 3H), 1.26ppm (m, 12H), 1.47ppm (m, 2H), 1.60ppm (m, 4H), 1.70ppm (m, 1H), 1.74ppm (m, 2H), 2.25ppm (m, 1H), 2.53ppm (m, 2H), 2.80ppm (m, 1H), 3.02ppm (m, 1H), 3.38ppm(s, 0.6H), 3.55ppm(s, 1.4H), 6.23ppm(br, 0.7H), 7.51ppm (br, 0.3H).

### Production Example 3

### Production of N-(4-hydroxy-2-methylphenyl)-3-oxododecanoyl amide (Compound 1b-1)

### (1) Synthesis of N-(4-hydroxy-2-methylphenyl)-3,3-ethyleneglycosyldodecanoyl amide

0.89g (3.44mmol) of 3,3-ethylene glycosyldodecanoyl acid obtained in Reference Example 1 and 0.85g (6.87mmol) of 4-amino-3-methylphenol were dissolved in 20mL of dichloromethane. 0.84g (6.87mmol) of dimethylaminopyridine, 0.98g (7.6mmol) of diisopropylethylamine, 1.05g (6.86mmol) of 1-hydroxy-1,2,3 triazole and 1.32g (6.87mmol) of 1-ethyl-3-dimethylaminopropyl carbodiimide were added to the solution, and the mixture was stirred at room temperature for 12 hours. The reaction liquid was concentrated under reduced pressure, and the residue was subjected to extraction with ethyl acetate. The obtained organic layer was washed with diluted hydrochloric acid and saturated saline, dried with magnesium sulfate, and concentrated under reduced pressure. The resulting residue was purified by silica gel chromatography (developing solvent: hexane/ethyl acetate=3/7) to obtain N-(4-hydroxy-2-methylphenyl)-3,3-ethyleneglycosyl dodecanoyl amide denoted by the following formula. 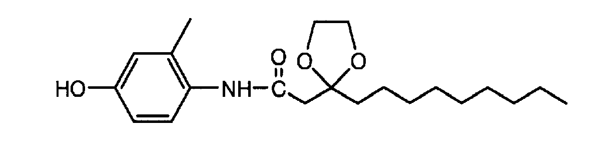 Quantity Yield: 0.97g (2.68mmol)
Percent Yield: 78%
¹H-NMR (CDCl₃, 500MHz); 0.88ppm (t, 3H), 1.23ppm (m, 12H), 1.37ppm (m, 2H), 1.72ppm (m, 2H), 2.37ppm (s, 3H), 3.99ppm (m, 4H), 6.70ppm (m, 2H), 7.43ppm (d, 1H), 9.04ppm (br, 1H).

### (2) Synthesis of N-(4-hydroxy-2-methylphenyl)-3-oxododecanoyl amide

15mL of trifluoroacetic acid was added to 0.97g (2.68mmol) of the N-(4-hydroxy-2-methylphenyl)-3,3-ethyleneglycosyldodecanoyl amide obtained in the foregoing process, and the mixture was stirred at room temperature for 18 hours. The reaction liquid was neutralized by a 5% sodium hydroxide aqueous solution, and the residue was subjected to extraction with ethyl acetate. The obtained organic layer was washed with saturated saline, dried with magnesium sulfate, and concentrated under reduced pressure. The resulting residue was purified by silica gel chromatography (developing solvent: ethyl acetate/methanol=3/7) to obtain N-(4-hydroxy-2-methylphenyl)-3-oxododecanoyl amide denoted by the following formula (Compound 1b-1). The Compound 1b-1 corresponds to the amide compound defined by Formula (1b) in which R¹ is a methyl group, R² is a hydroxyl group, and R is an n-nonyl group. 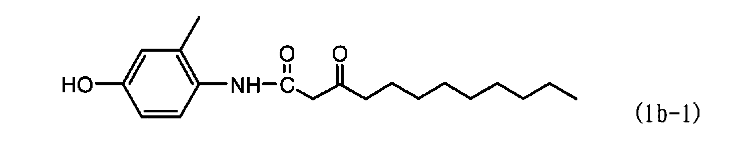 Quantity Yield: 0.81g (2.54mmol)
Percent Yield: 95%
¹H-NMR (CDCl₃, 500MHz); 0.90ppm (t, 3H), 1.18ppm (m, 12H), 1.65ppm (m, 2H), 2.37ppm (s, 3H), 2.60ppm (m, 2H), 3.61ppm (s, 2H), 6.70ppm (m, 2H), 7.63ppm (d, 1H), 8.94ppm (br, 1H).

### Production Example 4

### N-(3-hydroxypyridine-2-yl)-3-oxohexanoyl amide (Compound 1c-1)

### (1) Synthesis of N-(3-hydroxypyridine-2-yl)-3,3-ethyleneglycosyl hexanoyl amide

9.86g (56.7mmol) of 3,3-ethyleneglycosyl hexanoyl acid obtained in Reference Example 2 and 6.86g (62.4mmol) of 2-amino-3-hydroxypyridine were dissolved in 100mL of dichloromethane. 7.61g (62.4mmol) of dimethylaminopyridine, 9.51g (73.7mmol) of diisopropylethylamine, 11.94g (62.4mmol) of 1-ethyl-3-dimethylaminopropyl carbodiimide were added to the solution, and the mixture was stirred at room temperature for 12 hours. The reaction liquid was concentrated under reduced pressure, and the residue was subjected to extraction with ethyl acetate. The obtained organic layer was washed with saturated saline, dried with magnesium sulfate, and concentrated under reduced pressure. The resulting residue was purified by silica gel chromatography (developing solvent: hexane/ethyl acetate=6/4) to obtain N-(3-hydroxypyridine-2-yl)-3,3-ethyleneglycosyl hexanoylamide denoted by the following formula. 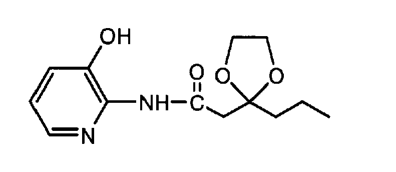 Quantity Yield: 9.05g (34.0mmol)
Percent Yield: 60%
¹H-NMR (CDCl₃, 500MHz); 0.90ppm (t, 3H), 1.41ppm (m, 2H), 1.78ppm (m, 2H), 4.05ppm (m, 4H), 7.05ppm (m, 1H), 7.17ppm (m, 1H), 7.74ppm (m, 1H), 9.55ppm (br, 1H).

### (2) Synthesis of N-(3-hydroxypyridine-2-yl)-3-oxohexanoylamide

5mL of trifluoroacetic acid was added to 6.56g (33.8mmol) of the N-(3-hydroxypyridine-2-yl)-3,3-ethyleneglycosyl hexanoylamide obtained in the foregoing process (1), and the mixture was stirred at room temperature for 18 hours. The reaction liquid was neutralized by a 5% sodium hydroxide aqueous solution, and the residue was subjected to extraction with ethyl acetate. The obtained organic layer was washed well with saturated saline, dried with magnesium sulfate, and concentrated under reduced pressure. The resulting residue was purified by silica gel chromatography (developing solvent: hexane/ethyl acetate=6/4) to obtain N-(3-hydroxypyridine-2-yl)-3-oxohexanoylamide denoted by the following formula (Compound 1c-1).

The Compound 1c-1 corresponds to the amide compound defined by Formula (1c) in which R³ is a hydroxyl group, and R is an n-nonyl group. 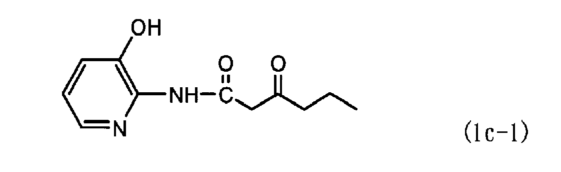 Quantity Yield: 5.7g(29.4mmol)
Percent Yield: 87%
¹H-NMR (CDCl₃, 500MHz); 0.97ppm (t, 3H), 1.69ppm (m, 2H), 2.60ppm (m, 2H), 3.66ppm (s, 2H), 7.15ppm (m, 1H), 7.37ppm (m, 1H), 7.94ppm (m, 1H), 10.05ppm (br, 1H).

### Experiment Example 1

### Evaluation test for an effect for removing biofilms formed by Pseudomonas aeruginosa

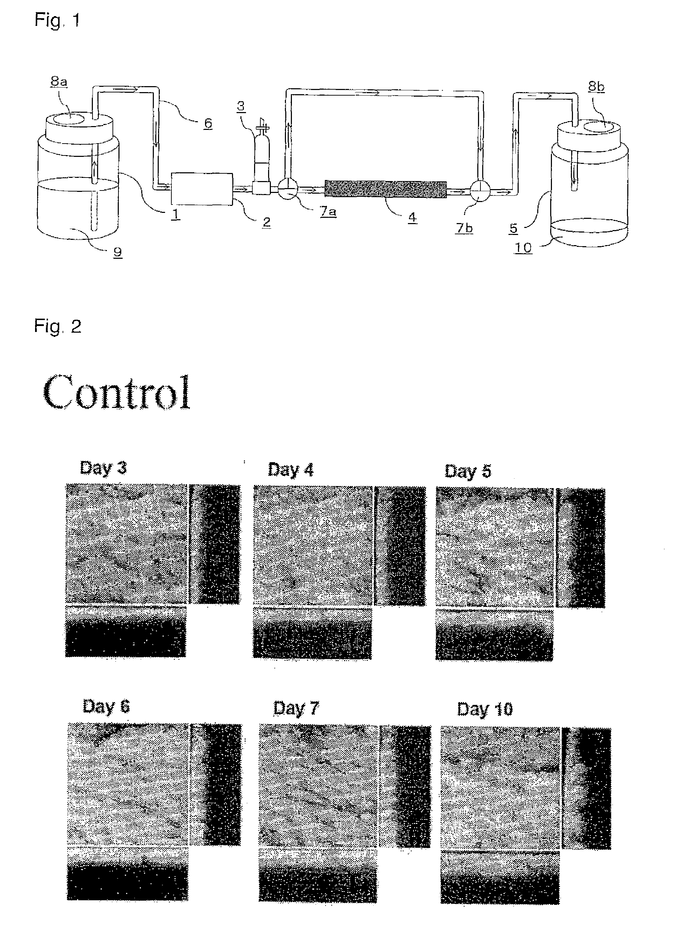

Each of the amide compounds (Compound 1a-1, 1a-2, 1b-1 and 1c-1) produced in Production Examples 1 to 4 was evaluated for its biofilm removal effect, using the flow cell system shown in FIG. 1.

### Flow Cell System

A culture medium bottle (1), a peristaltic pump (2), a glass cell (4) and a waste fluid bottle (5) are connected via a silicon tube (6) so that a culture medium (9) is supplied to the glass cell from the culture medium bottle (1) using the peristaltic pump (2). The system includes an air removal section (3) between the peristaltic pump (2) and a turncock (7a), for removing the entrained air. All tools in the flow cell system are sterilized using gamma-rays or an autoclave.

As specifically described later, the effect for removing biofilms is examined in the following manner. Bacteria are cultured in a glass cell (4) to grow a biofilm on the inner wall of the cell, and a test liquid containing one of the amide compounds is applied to the biofilm. Then the condition of the biofilm is observed to evaluate the biofilm-removing effect of the compound using a fluorescence confocal microscope (Leica TCS SP2: product of Leica).

### Materials

### Bacteria

The bacteria was prepared by inserting a pTdk-LVAgfp plasmid into a Pseudomonas aeruginosa PAO1-strain using the electroportion method so that the Pseudomonas aeruginosa PAO1-strain is transformed to express Green Fluorescent Protein (hereinafter referred to as "gfp expression PAO1-strain") (see Teresa R. et al., Applied and Environmental Microbiology, Apr. 2001, p.1865-1873).

### Culture Medium

A 30mM glucose-containing FAB culture medium was used for preincubation, and a 0.3mM glucose-containing FAB culture medium was used for main cultivation.

### Glucose-Containing FAB Culture Medium

The glucose-containing FAB culture medium was prepared by adding 200µg/mL of carbenicillin to an aqueous solution containing 30mM glucose or 0.3mM glucose, 15mM ammonium sulfate, 33.7mM disodium hydrogenphosphate, 22.1mM dipotassium hydrogenphosphate, 51.7mM sodium chloride, 0.47mM magnesium chloride, 0.08mM calcium chloride and the following 0.1% trace metal solution.

### Trace Metal Solution

An aqueous solution containing 1.16mM calcium sulfate dihydrate, 0.72mM iron sulfate heptahydrate, 0.08mM manganous sulfate monohydrate, 0.08mM copper sulfate pentahydrate, 0.07mM zinc sulfate heptahydrate, 0.04mM cobalt sulfate heptahydrate, 0.04mM sodium permanganate monohydrate, and 0.08mM boric acid.

### Bacterial Suspension

A gfp expression PAO1-strain was inoculated in a 30mM glucose-containing FAB culture medium, and was subjected to shake culture at 37°C in an isothermal tank overnight. The obtained overnight culture liquid was diluted by a 30mM glucose-containing FAB culture medium to OD₅₉₀ = 0.1.

### Test Liquid and Control Test Liquid

### Test Liquid

The test liquid was prepared by dissolving 100µM of each of the Compounds 1a-1, 1a-2, and 1b-1 and 1c-1 according to Production Examples 1 to 4 and 100µM of a comparative compound 1 as a comparative example denoted by the following formula to dimethyl sulfoxide (DMSO), and supplying the resulting DMSO solutions to a 0.3mM glucose-containing FAB culture medium so that the concentration of each compound becomes 0.1%(v/v).

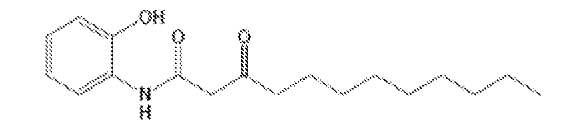

### Control Test Liquid

A DMSO not containing the compound was used as a control test liquid.

### Test Method

The flow cell system was filled with hydrous ethanol (70 volume%), and was allowed to stand for at least 12 hours to sterilize the system. Then, air having been filtrated by a membrane filter (8a) was supplied into the flow cell system using the peristaltic pump (2) to dry the system. Thereafter, the system was filled with a 0.3mM glucose-containing FAB culture medium. 500µL of a bacterial suspension was injected into the glass cell (4) from the upper surface, and the glass cell was sealed by turning off the three-way turncock (7a,7b). The cell as such was allowed to stand for an hour at room temperature.

After the static culture, two of the three-way turncocks (7a,7b) were turned on, so as to supply each test liquid and control test liquid at a flow rate of 200µL per minute. With a fluorescence confocal microscope, the process of biofilm deposition by the Pseudomonas aeruginosa (gfp expression PAO1-strain) adhered to the inner wall of the glass cell was observed three-dimensionally from above to observe changes over time (after 3 days, 4 days, 5 days, 6 days, 7 days, and 10 days). Note that the test liquid and the control test liquid were replaced with fresh liquids for every 36 hours.

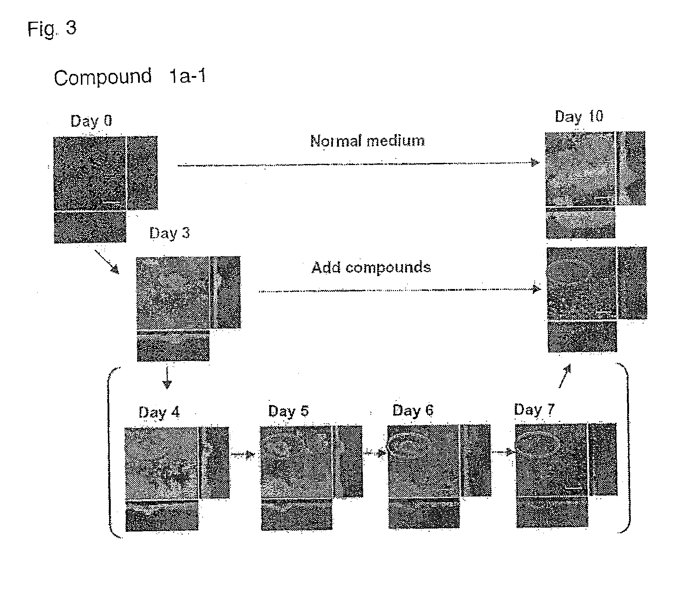

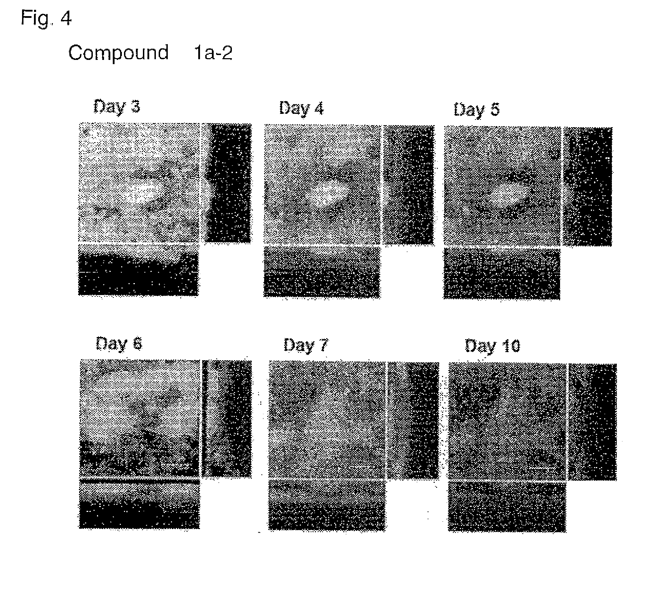

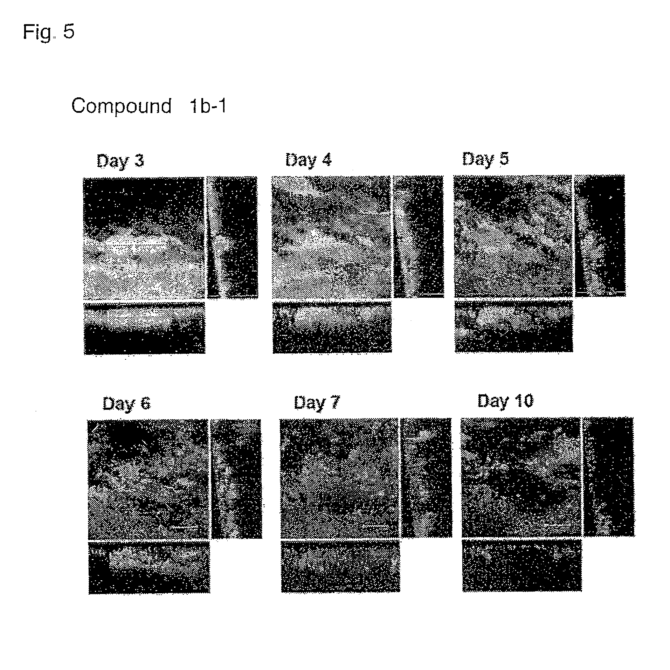

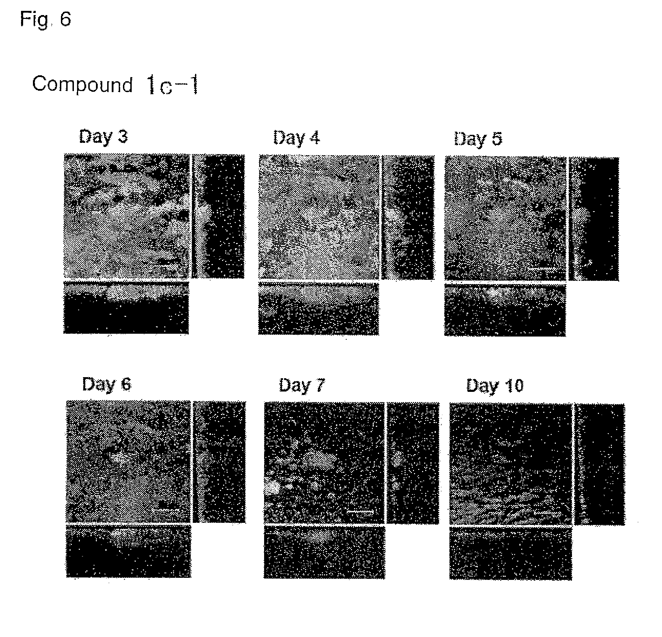

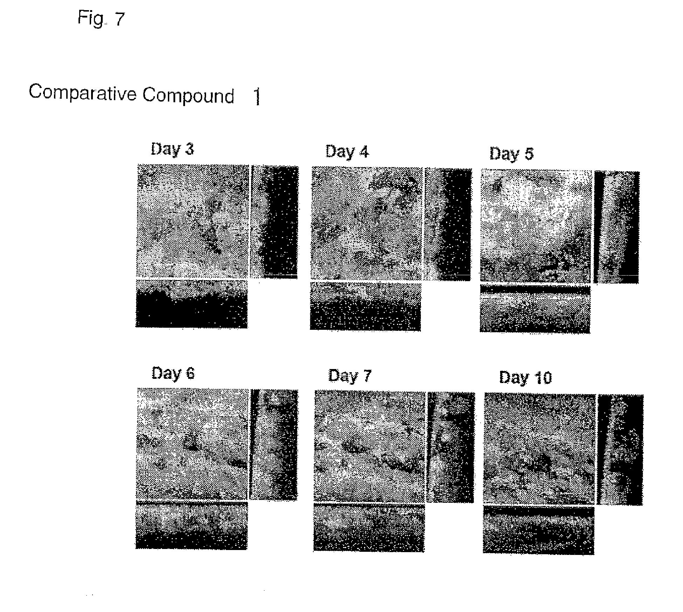

FIG. 2 shows a biofilm condition in the experiment for the control test liquid (control test). FIG. 3 shows a comparison result between the biofilm condition in the experiment for the control test liquid (control test) and the biofilm condition in the experiment for the test liquid (Compound 1a-1). FIGS. 4, 5, 6 and 7 show the biofilm conditions in the experiments for different test liquids: Compounds 1a-2, 1b-1, 1c-1, and Comparative Compound 1, respectively. FIGS. 2 to 7 show three-dimentional conditions of biofilm formed by Pseudomonas aeruginosa (gfp expression PAO1-strain) adhered to the inner wall of the glass cell in the respective experiments, with the front views of the inner wall of the glass cell and the cross-sectional (vertical and horizontal) views of the glass cells.

According to those results, in the control test using the control test liquid, it was observed that Pseudomonas aeruginosa (gfp expression PAO1-strain) formed a huge, round, thick biofilm that further grew even larger after ten days' cultivation. In contrast, in the test using the test liquid containing Compound 1a-2, 1b-1, or 1c-1, the gradual disappearance of the deposited biofilm with time was observed. Also, the gradual exfoliation of the deposited biofilm from the cell wall, and the flowing flakes of biofilm exfoliation in the cell were also visually observed. Meanwhile, in the test using the test liquid containing Comparative Compound 1, a similar biofilm formation to that of the control test using the control test liquid was observed.

This result showed that Compounds 1a-1, 1a-2, 1b-1 and 1c-1 prepared in Production Examples 1 to 4 are effective for removing deposited biofilms.

### Experiment Example 2

### Evaluation test for effectiveness in removing biofilms formed by periodontitis pathogenic bacteria

The same experiment as in Experiment Example 1 was conducted except that periodontitis pathogenic bacteria Porphyromonas gingivalis 381-strain (clinical bacteria) was used in place of Pseudomonas aeruginosa PAO1-strain. The state of the biofilm was observed with an optical microscope, and nearly the entire biofilm was removed in 10 days by Compound 1a-1, Compound 1a-2, Compound 1b-1 or Compound 1c-1. The gradual exfoliation of the deposited biofilm from the cell wall, and the flowing flakes of biofilm exfoliation in the cell were also visually observed.

### EFFECT OF THE INVENTION

The compound and the biofilm remover of the present invention are capable of stripping off biofilms, thereby solving various defects caused by biofilms formed by microorganisms. The compound and the biofilm remover of the present invention have a particular characteristic of stripping off already-formed biofilms, thereby ensuring significant effects for treating intractable biofilm infections. With this characteristic, the compound and the biofilm remover of the present invention will greatly contribute to a complete cure of the biofilm infections.

For example, Pseudomonas aeruginosa, which exists everywhere in the natural environment, requires a small amount of organic matter and moisture to breed and grow into a biofilm. Therefore, Pseudomonas aeruginosa often causes in-hospital infections, substituted microbism, opportunistic infection, and sanitary defects in water pipes or water storage tanks.

Further, periodontitis pathogenic bacteria forms a biofilm called plaque, which causes oral odor or intraoral diseases such as periodontitis or alveolar pyorrhea. It has been publicly known that the removal of plaque is important in intraoral sanitation or for the treatment of intraoral diseases.

However, as described above, biofilms are resistant to disinfectants to a certain extent.

In view of this problem, the biofilm remover of the present invention facilitates the removal of biofilms formed by Pseudomonas aeruginosa in pipes or water storage tanks in hospital facilities or private residences, preventing in-hospital infections or other various defects caused by biofilms, thereby improving environmental sanitation. The biofilm remover of the present invention is also capable of stripping off biofilms on teeth, gingiva or intraoral dental materials, formed by periodontitis pathogenic bacteria, thereby effectively preventing or treating gingival disease such as periodontitis or alveolar pyorrhea, intraoral diseases such as stomatitis, as well as reducing oral odor.

## Claims

1. A biofilm remover containing, as an active ingredient, an amide compound denoted by General Formula (1) or salt thereof, 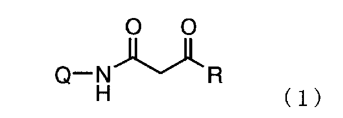 wherein R is a C₁₋₁₁ alkyl group, and Q is a substituent denoted by the following Formulas (Q1), (Q2) or (Q3), 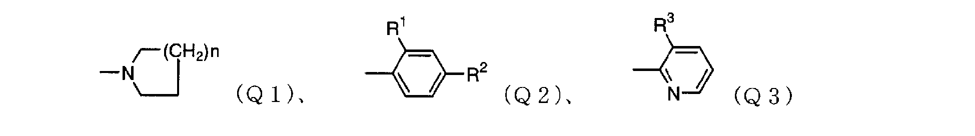 wherein, in Formula (Q1), n is an integer ranging from 0 to 4; in Formula (Q2), R¹ is a C₁₋₄ alkyl group, and R² is a hydroxyl group or carbamoyl group; in Formula (Q3), R³ is a hydrogen atom, hydroxyl group or carbamoyl group.

2. A biofilm remover according to claim 1, wherein, in General Formula (1), Q is a substituent denoted by Formula (Q1) wherein n is 1 or 2.

3. A biofilm remover according to claim 1, wherein, in General Formula (1), R is a C₇₋₁₀ alkyl group, and Q is a substituent denoted by Formula (Q1) wherein n is 1 or 2.

4. A biofilm remover according to claim 1, wherein, in General Formula (1), R is an n-nonyl group, and Q is a substituent denoted by Formula (Q1) wherein n is 1 or 2.

5. A biofilm remover according to claim 1, wherein, in General Formula (1), Q is a substituent denoted by Formula (Q2) wherein R¹ is a C₁₋₄ alkyl group and R² is a hydroxyl group or carbamoyl group.

6. A biofilm remover according to claim 1, wherein, in General Formula (1), Q is a substituent denoted by Formula (Q2) wherein R¹ is a methyl group and R² is a hydroxyl group or carbamoyl group.

7. A biofilm remover according to claim 1, wherein, in General Formula (1), R is a C₇₋₁₀ alkyl group, and Q is a substituent denoted by Formula (Q2) wherein R¹ is methyl group, and R² is a hydroxyl group or carbamoyl group.

8. A biofilm remover according to claim 1, wherein, in General Formula (1), R is an n-nonyl group, and Q is a substituent denoted by Formula (Q2) wherein R¹ is a methyl group and R² is a hydroxyl group.

9. A biofilm remover according to claim 1, wherein, in General Formula (1), Q is a substituent denoted by Formula (Q3) wherein R³ is a hydroxyl group.

10. A biofilm remover according to claim 1, wherein, in General Formula (1), R is a C₁₋₄ alkyl group, and Q is a substituent denoted by Formula (Q3) wherein R³ is a hydroxyl group.

11. A biofilm remover according to claim 1, wherein, in General Formula (1), R is an n-propyl group, and Q is a substituent denoted by Formula (Q3) wherein R³ is a hydroxyl group.

12. A biofilm remover according to any one of claims 1 to 11, wherein the biofilm is film formed by Pseudomonas aeruginosa.

13. A biofilm remover according to any one of claims 1 to 11, wherein the biofilm is film formed by periodontitis pathogenic bacteria.

14. An oral composition containing the biofilm remover according to claim 13.

15. An amide compound denoted by General Formula (1b) or salt thereof, 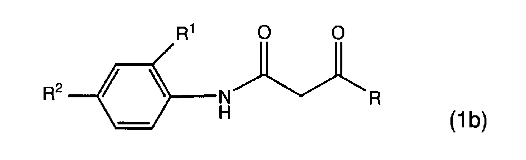 wherein R is a C₁₋₁₁ alkyl group, R¹ is a C₁₋₄ alkyl group, and R³ is a hydroxyl group or carbamoyl group.

16. An amide compound or salt thereof according to claim 15, wherein, in General Formula (1b), R¹ is a methyl group.

17. An amide compound or salt thereof according to claim 15, wherein, in General Formula (1b), R² is a hydroxy group.

18. An amide compound or salt thereof according to claim 15, wherein, in General Formula (1b), R is a C₇₋₁₀ alkyl group.

19. An amide compound or salt thereof according to claim 15, wherein, in General Formula (1b), R is an n-nonyl group, R¹ is a methyl group, and R² is a hydroxy group.

20. An amide compound denoted by General Formula (2) or salt thereof, 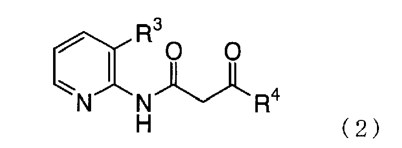 wherein R³ is a hydrogen atom, hydroxyl group or carbamoyl group, and R⁴ is a C₁₋₄ alkyl group.

21. An amide compound or salt thereof according to claim 20, wherein, in General Formula (2), R³ is a hydroxyl group.

22. An amide compound or salt thereof according to claim 20, wherein, in General Formula (2), R⁴ is an n-propyl group.

23. An amide compound or salt thereof according to claim 20, wherein, in General Formula (2), R³ is a hydroxyl group, and R⁴ is an n-propyl group.
